# EUROPEAN PATENT SPECIFICATION

(11) **EP 4 324 814 B1**
(45) Date of publication and mention of the grant of the patent: **24.12.2025**
(21) Application number: 22788064.8
(22) Date of filing: 30.03.2022
(51) Int. Cl.: C07C 29/149, C07C 31/27, C07C 67/31, B01J 21/04, B01J 23/80, B01J 23/00

(54) **METHOD FOR PRODUCING DIMETHANOL COMPOUND HAVING NORBORNANE SKELETON**
VERFAHREN ZUR HERSTELLUNG EINER DIMETHANOLVERBINDUNG MIT NORBORNANGERÜST
PROCÉDÉ DE FABRICATION DE COMPOSÉ DIMÉTHANOL POSSÉDANT UN SQUELETTE DE NORBORNANE

(30) Priority: 12.04.2021 JP 2021067275
(43) Date of publication of application: 21.02.2024
(73) Proprietor: MITSUBISHI GAS CHEMICAL COMPANY, INC., Chiyoda-ku Tokyo 100-8324 (JP)
(72) Inventor: KITAMURA, Mitsuharu, Tokyo 125-8601 (JP); NISHIDA, Takeru, Niigata-shi, Niigata 950-3112 (JP); NAKANISHI, Yuta, Niigata-shi, Niigata 950-3112 (JP); IDA, Hiromichi, Niigata-shi, Niigata 950-3112 (JP); KOSAKI, Hideaki, Niigata-shi, Niigata 950-3121 (JP)
(74) Representative: Vossius & Partner Patentanwälte Rechtsanwälte mbB
(86) International application number: PCT/JP2022/016222
(87) International publication number: WO 2022/220139

(56) References cited:
- EP-A1- 3 202 815
- WO-A1-2013/161594
- WO-A1-2015/147242
- WO-A1-2016/052370
- WO-A1-2020/213389
- JP-A- H0 782 190
- JP-A- S63 152 338
- US-A1- 2017 088 504

## Description

### Technical Field

The present invention relates to a production method for producing a dimethanol compound having a norbornane skeleton.

### Background Art

A dimethanol compound having a norbornane skeleton is known to exhibit excellent features when used as an adhesive or a resin raw material. As for a method for producing such a dimethanol compound having a norbornane skeleton, for example, Patent Literature 1 describes that the dimethanol compound having a norbornane skeleton is obtained through the hydrogenation reaction of a corresponding ester compound.

### Citation List

### Patent Literature

Patent Literature 1: International Publication No. WO 2015/147242

### Summary of Invention

### Technical Problem

Hydrogenation reaction is typically carried out by reacting a compound having ester with a hydrogen gas using a catalyst containing copper oxide and a reaction solvent. In Patent Literature 1, after addition of a substrate ester compound, a toluene solvent, and a copper oxide catalyst, the reaction is performed at a reaction temperature of 215°C at a reaction pressure of 10 MPa for a reaction time of 8 hours. However, the amount of the catalyst is as large as 20 wt% based on the substrate, and the amount of the toluene solvent used is as large as 300 wt% based on the substrate. Furthermore, the produced dimethanol compound has been found to be low soluble in the toluene solvent and thus precipitated as a paste-like product of the catalyst, the dimethanol compound, and an unreacted ester compound in the toluene solvent. In this case, a catalyst filtration/separation can be carried out by diluting the product with a methanol solvent after the completion of reaction. However, it has been found that, in addition to the dimethanol compound which is a target compound, approximately 1% of an intermediate ester compound remains in the reaction liquid. In the case of using the target compound as a polymer raw material, the intermediate that could be remained in the target compound tends to inhibit reaction. Furthermore, the target compound and the intermediate both have a high viscosity and a high boiling point and are therefore difficult to separate by simple distillation or thin-membrane distillation. Although it is possible to use distillation purification at a large number of stages for the separation of the intermediate from the target compound, this method would compromise yield and therefore cannot be said to be an economic method.

Thus, the technique of Patent Literature 1 leaves a room for improvement from the viewpoint of suppressing the production of the intermediate.

Meanwhile, use of an alcohol-based solvent could solve the problem that a paste-like product is precipitated in a solvent. However, methanol or ethanol has a partial pressure due to the influence of a high reaction temperature of hydrogenation reaction and tends to lengthen a reaction time because a hydrogen gas concentration in a gas phase is lowered. Thus, this method cannot be said to be an economic method.

In Patent Literature 1, cyclohexanol, which is used as a solvent, can avoid lowering a hydrogen gas concentration as described above. However, it has been found to partially cause transesterification reaction with an intermediate and easily produce a transesterification reaction product as a by-product in a reaction liquid. In this case, as in the aforementioned case of the intermediate, reaction into a polymer tends to be inhibited, and the target compound and the by-product both have a high viscosity and a high boiling point and are therefore difficult to separate by simple distillation or thin-membrane distillation. Use of distillation purification at a large number of stages for the separation would compromise yield. Therefore, this method cannot be said to be an economic method.

Thus, the technique of Patent Literature 1 also leaves a room for improvement from the viewpoint of suppressing the production of the by-product.

The present invention has been made in light of the problems described above. An object of the present invention is to provide a method for producing a dimethanol compound which can suppress both the production of an intermediate and the production of a by-product in the production of the dimethanol compound through hydrogenation reaction.

### Solution to Problem

The present inventors have found that the problem can be solved by using a predetermined alcohol as a solvent for use in hydrogenation reaction in the production of a dimethanol compound having a norbornane skeleton, and thus have completed the present invention.

Specifically, the present invention is as follows.

A production method for producing a target compound represented by the following formula (1), the production method comprising:
a step (A) of subjecting a mixed liquid comprising a starting compound represented by the following formula (2) and a solvent to a hydrogenation reduction in a presence of a catalyst having hydrogenation ability,
wherein the solvent is a linear or branched secondary alcohol or tertiary alcohol: 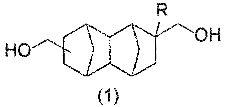 wherein R represents H, CH₃, or C₂H₅, 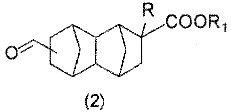 wherein R represents H, CH₃, or C₂H₅, and R₁ represents CH₃, C₂H₅, C₃H₇, C₄H₉.

In the production method, a boiling point of the solvent may be 100 to 230°C.

In the production method, the solvent may be represented by the following formula (a): 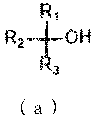 wherein R₁ represents H or CH₃, R₂ represents CH₃, C₂H₅, C₃H₇, or C₄H₉, and R₃ represents C₃H₇, C₄H₉, C₅H₁₁, C₆H₁₃, C₇H₁₅, C₈H₁₇, C₉H₁₉, C₁₀H₂₁, C₁₁H₂₃, or C₁₂H₂₅.

In the production method, a content of an intermediate represented by the following formula (3) in the target compound may be 0.5% by mass or less: 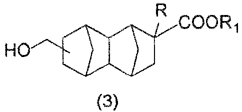 wherein R represents H, CH₃, or C₂H₅, and R₁ represents CH₃, C₂H₅, C₃H₇, or C₄H₉.

In the production method, a content of a by-product represented by the following formula (4) in the target compound may be 0.5% by mass or less: 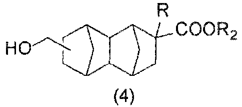 wherein R represents H, CH₃, or C₂H₅, and R₂ represents C₄H₉, C₅H₁₁, C₆H₁₃, C₇H₁₅, C₈H₁₇, C₉H₁₉, C₁₀H₂₁, C₁₁H₂₃, C₃₂H₂₅, C₁₃H₂₇, or C₁₄H₂₉.

In the production method, the solvent may be 2-octanol and/or tetrahydrolinalool.

In the production method, in the step (A), the catalyst may be activated at a first temperature and a first pressure, and after the activation, the starting compound may be hydrogenated at a second temperature and a second pressure.

The second pressure may be higher than 5 MPa and 20 MPa or lower.

The second pressure may be higher than 5 MPa and 8 MPa or lower, and wherein the solvent may optionally be a linear or branched tertiary alcohol, for example, tetrahydrolinalool.

### Advantageous Effects of Invention

The production method according to the present invention can provide a production method which can suppress both the production of an intermediate and the production of a by-product in the production of a dimethanol compound through hydrogenation reaction. Description of Embodiments

Hereinafter, an embodiment for carrying out the present invention (hereinafter, also referred to as the "present embodiment") will be described in detail. The present embodiment given below is an example for explaining the present invention and does not intend to limit the present invention to the contents given below. The present invention can be carried out through appropriate changes or modifications without departing from the scope of the present invention.

### <Method for producing dimethanol compound having norbornane skeleton>

The production method of the present embodiment is a method for producing a target compound represented by the following formula (1) (i.e., a dimethanol compound having a norbornane skeleton, represented by the formula (1); hereinafter, also simply referred to as a "target compound"), the production method comprising a step (A) of subjecting a mixed liquid containing a starting compound represented by the following formula (2) and a solvent to hydrogenation reduction in the presence of a catalyst having hydrogenation ability, wherein the solvent is a linear or branched secondary alcohol or tertiary alcohol. 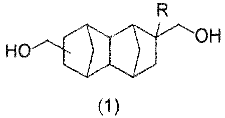 wherein R represents H, CH₃, or C₂H₅, 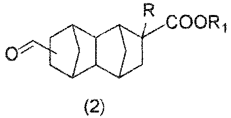 wherein R represents H, CH₃, or C₂H₅, and R₁ represents CH₃, C₂H₅, C₃H₇, C₄H₉.

The production method of the present embodiment is configured as described above and as such, can suppress both the production of an intermediate and the production of a by-product in the production of a dimethanol compound through hydrogenation reaction. The target compound can be preferably used as a paint additive, an adhesive, a resin raw material, or the like.

A synthesis route for producing the target compound can adopt, for example, but not limited to, a synthesis route shown in the following formula (I) using dicyclopentadiene or cyclopentadiene and olefin having a functional group as starting materials: 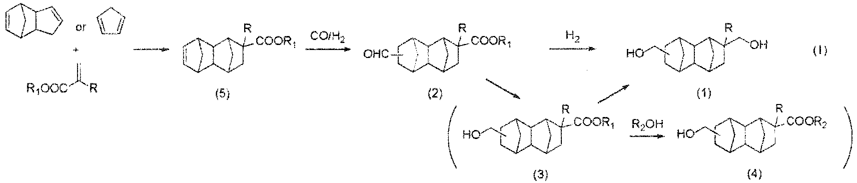 wherein R represents H, CH₃, or C₂H₅, R₁ represents CH₃, C₂H₅, C₃H₇, or C₄H₉, and R₂ represents an organic group in a linear or branched secondary alcohol or tertiary alcohol and preferably represents C₄H₉, C₅H₁₁, C₆H₁₃, C₇H₁₅, C₈H₁₇, C₉H₁₉, or C₁₀H₂₁.

The formula (3) in the formula (I) represents a synthetic intermediate capable of being entrained with the target compound. Hereinafter, this synthetic intermediate is also simply referred to as an "intermediate" for the sake of convenience of description. The formula (4) in the formula (I) represents a by-product capable of being entrained with the target compound. Hereinafter, this by-product is also simply referred to as a "by-product" for the sake of convenience of description.

### [Monoolefin having 14 to 19 carbon atoms represented by formula (5)]

The monoolefin having 14 to 19 carbon atoms represented by the following formula (5) according to the present embodiment can be produced through the Diels-Alder reaction of olefin having a functional group with dicyclopentadiene: 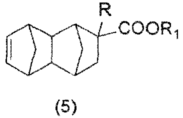 wherein R represents H, CH₃, or C₂H₅, and R₁ represents CH₃, C₂H₅, C₃H₇, or C₄H₉.

Examples of the olefin having a functional group for use in the Diels-Alder reaction include, but are not particularly limited to, methyl methacrylate, ethyl methacrylate, propyl methacrylate, butyl methacrylate, methyl acrylate, ethyl acrylate, propyl acrylate, and butyl acrylate. Among them, methyl methacrylate, ethyl methacrylate, methyl acrylate, and ethyl acrylate are preferred.

The dicyclopentadiene for use in the Diels-Alder reaction of the present embodiment preferably has a high purity and preferably has a lower content of impurities such as butadiene and isoprene. The purity of the dicyclopentadiene is preferably 90% or more, more preferably 95% or more. It is known that dicyclopentadiene is depolymerized into cyclopentadiene (so-called monocyclopentadiene) under heating conditions. Therefore, cyclopentadiene may be used instead of the dicyclopentadiene. It is considered that the monoolefin having 14 to 19 carbon atoms represented by the formula (5) is substantially produced via monoolefin having 9 to 14 carbon atoms represented by the formula (6) given below (first-stage Diels-Alder reaction product). It is also considered that the produced monoolefin of the following formula (6) undergoes, as a new parent diene compound (dienophile), Diels-Alder reaction (second-stage Diels-Alder reaction) with cyclopentadiene (diene) present in the reaction system to produce the monoolefin having 14 to 19 carbon atoms represented by the formula (5): 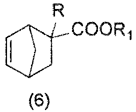 wherein R represents H, CH₃, or C₂H₅, and R₁ represents CH₃, C₂H₅, C₃H₇, or C₄H₉.

In the presence of cyclopentadiene in the reaction system, the Diels-Alder reaction at two sages tends to proceed efficiently. Therefore, the reaction temperature of the Diels-Alder reaction is preferably 100°C or higher, more preferably 120°C or higher, further preferably 130°C or higher. On the other hand, it is preferred to perform the reaction at a temperature of 250°C or lower for suppressing the production of a high-boiling substance as a by-product. Hydrocarbons, alcohols, esters, or the like may be used as reaction solvents, and aliphatic hydrocarbons having 6 or more carbon atoms, cyclohexane, toluene, xylene, ethylbenzene, mesitylene, propanol, butanol, or the like is suitable.

Diverse reaction schemes such as a batch system using a tank-type reactor or the like, a semi-batch system in which a substrate or a substrate solution is supplied to a tank-type reactor under reaction conditions, and a continuous distribution system in which substrates are distributed to a tube-type reactor under reaction conditions can be adopted as reaction schemes of the Diels-Alder reaction.

The reaction product (monoolefin having 14 to 19 carbon atoms represented by the formula (5)) obtained through the Diels-Alder reaction may be directly used as a starting material for subsequent hydroformylation reaction or may be subjected to the next step after being purified by a method such as distillation, extraction, or crystal precipitation. The monoolefin having 14 to 19 carbon atoms represented by the formula (5) is not limited to those synthesized as mentioned above, and a commercially available product, if obtainable, may be used in the production method of the present embodiment.

### [Starting compound represented by formula (2)]

The starting compound represented by the formula (2) in the formula (I) can be produced, for example, by subjecting the monoolefin having 14 to 19 carbon atoms represented by the formula (5) and carbon monoxide and hydrogen gases to hydroformylation reaction in the presence of a rhodium compound and an organic phosphorus compound.

The rhodium compound for use in the hydroformylation reaction is not particularly limited, and, for example, a compound that forms a complex with an organic phosphorus compound and exhibits hydroformylation activity in the presence of carbon monoxide and hydrogen, can be used. A precatalyst such as dicarbonyl(acetylacetonato)rhodium (hereinafter, also referred to as "Rh(acac)(CO)₂") , Rh₂O₃, Rh₄(CO)₁₂, Rh₆(CO)₁₆, or Rh (NO₃)3; may be introduced together with an organic phosphorus compound into a reaction mixture to form a rhodium-metal carbonyl hydride-phosphorus complex having catalytic activity in a reaction container, or a rhodium-metal carbonyl hydride-phosphorus complex may be prepared in advance and introduced into a reactor. In the present embodiment, it is preferred that Rh(acac)(CO)₂ should be reacted with an organic phosphorus compound in the presence of a solvent and then introduced together with an excess of an organic phosphorus compound into a reactor to form a rhodium-organic phosphorus complex having catalytic activity, which is used in the hydroformylation reaction.

The amount of the rhodium compound used in the hydroformylation reaction is preferably 0.1 to 60 µmol, more preferably 0.1 to 30 µmol, further preferably 0.2 to 20 µmol, still further preferably 0.5 to 10 µmol, based on 1 mol of the monoolefin having 14 to 19 carbon atoms represented by the formula (5) which is a substrate of the hydroformylation reaction. When the amount of the rhodium compound used is less than 60 µmol based on 1 mol of the monoolefin having 14 to 19 carbon atoms, rhodium catalyst cost can be reduced without providing recovery and recycling facilities of a rhodium complex. Therefore, economic burdens associated with recovery and recycling facilities can be reduced.

In the hydroformylation reaction, examples of the organic phosphorus compound that forms a catalyst of the hydroformylation reaction with the rhodium compound include phosphine represented by the general formula P(-R₁)(-R₂)(-R₃) and phosphite represented by the general formula P(-OR₁)(-OR₂)(-OR₃). In these general formulas, specific examples of R₁, R₂, and R₃ include an aryl group optionally substituted by an alkyl group or an alkoxy group having 1 to 4 carbon atoms, and an alicyclic alkyl group optionally substituted by an alkyl group or an alkoxy group having 1 to 4 carbon atoms. Triphenylphosphine or triphenyl phosphite is suitably used. The amount of the organic phosphorus compound used is preferably 300 times to 10000 times, more preferably 500 times to 10000 times, further preferably 700 times to 5000 times, still further preferably 900 times to 2000 times the mol of a rhodium atom in the rhodium compound. When the amount of the organic phosphorus compound used is 300 or more times the mol of a rhodium atom, a rhodium-metal carbonyl hydride-phosphorus complex as a catalytically active substance contributes to stability. As a result, reaction efficiency tends to be improved. When the amount of the organic phosphorus compound used is 10000 or less times the mol of a rhodium atom, economic performance tends to be improved from the viewpoint of cost required for the organic phosphorus compound.

The hydroformylation reaction may be performed without the use of a solvent and can be preferably carried out by using a solvent inert to the reaction. The solvent is not particularly limited as long as the solvent dissolves the monoolefin having 14 to 19 carbon atoms represented by the formula (5), the dicyclopentadiene or the cyclopentadiene, the rhodium compound, and the organic phosphorus compound. Specific examples thereof include hydrocarbons such as aliphatic hydrocarbon, alicyclic hydrocarbon, and aromatic hydrocarbon, esters such as aliphatic ester, alicyclic ester, and aromatic ester, alcohols such as aliphatic alcohol and alicyclic alcohol, and other solvents such as aromatic halide. Among them, hydrocarbons are preferred. Among the hydrocarbons, alicyclic hydrocarbon and aromatic hydrocarbon are preferred.

A temperature at which the hydroformylation reaction is performed is preferably 40°C to 160°C, more preferably 80°C to 140°C. When the reaction temperature is 40°C or higher, a sufficient reaction rate is obtained and prevents the starting material monoolefin from remaining. When the reaction temperature is 160°C or lower, the formation of by-products derived from the starting material monoolefin or a reaction product is suppressed. This tends to be able to prevent reduction in reaction performance.

In the case of performing the hydroformylation reaction, the reaction is performed under pressurization with carbon monoxide (hereinafter, also referred to as "CO") and hydrogen (hereinafter, also referred to as "H₂") gases. The CO and H₂ gases may be each independently introduced into the reaction system or may be introduced as a mixed gas prepared in advance into the reaction system. The molar ratio between the CO and H₂ gases (= CO/H₂) to be introduced into the reaction system is preferably 0.2 to 5, more preferably 0.5 to 2, further preferably 0.8 to 1.2. When the molar ratio between the CO and H₂ gases falls within the range described above, the reaction activity of the hydroformylation reaction and selectivity to target aldehyde tend to be improved. The amount of the CO and H₂ gases introduced into the reaction system decreases as the reaction proceeds. Therefore, use of a mixed gas of CO and H₂ prepared in advance may facilitate reaction control.

The reaction pressure of the hydroformylation reaction is preferably 1 to 12 MPa, more preferably 1.2 to 8 MPa, further preferably 1.5 to 5 MPa. When the reaction pressure is 1 MPa or higher, a sufficient reaction rate is easily obtained and tends to be able to prevent the starting material monoolefin from remaining. When the reaction pressure is 12 MPa or lower, an expensive facility excellent in pressure resistance is no longer necessary. This tends to be economically advantageous. Particularly, in the case of performing the reaction in a batch system or a semi-batch system which involves depressurization by the discharge of the CO and H₂ gases after the completion of reaction, a lower pressure causes a smaller loss of the CO and H₂ gases. This tends to be economically advantageous.

The reaction scheme in performing the hydroformylation reaction is preferably batch reaction or semi-batch reaction. The semi-batch reaction can be performed by adding the rhodium compound, the organic phosphorus compound, and the solvent to a reactor, creating the aforementioned reaction conditions through pressurization with CO/H₂ gases, heating, or the like, and then supplying the starting material monoolefin or its solution to the reactor.

The reaction product obtained through the hydroformylation reaction may be directly used as a starting material for subsequent reduction reaction or may be subjected to the next step after being purified by for example, distillation, extraction, or crystal precipitation. The starting compound represented by the formula (2) is not limited to those synthesized as mentioned above, and a commercially available product, if obtainable, may be used in the production method of the present embodiment.

### [Step (A)]

The production method of the present embodiment comprises a step (A) of subjecting a mixed liquid containing a starting compound represented by the formula (2) and a solvent to hydrogenation reduction in the presence of a catalyst having hydrogenation ability (hereinafter, also referred to as a "hydrogenation catalyst"). In the present embodiment, a linear or branched secondary alcohol or tertiary alcohol is used as the solvent in this step (A). By use of this solvent, not only is the target compound obtained from the starting compound, but both the formation of the intermediate and the formation of the by-product can be suppressed. Although the reason why use of the solvent according to the present embodiment can suppress the formation of both the intermediate and the by-product is not necessarily evident, this is presumably because the secondary alcohol and/or tertiary alcohol, either of which is linear or branched, is unlikely to cause transesterification reaction responsible for the by-product owing to moderate steric hindrance and, even if causing this reaction, facilitates forming the target product through subsequent hydrogenation. However, this inference does not intend to limit the mechanism of action of the present embodiment to those described above.

Use of the secondary alcohol and/or tertiary alcohol, either of which is linear or branched, as the solvent in the step (A) is of significance for the reduction reaction according to the present embodiment. If aliphatic hydrocarbons, alicyclic hydrocarbons, or aromatic hydrocarbons are used in hydrogenation reaction, the produced dimethanol compound is difficult to dissolve in the solvent and tends to be precipitated as a paste-like product of the catalyst, the dimethanol compound, and an unreacted ester compound in the solvent. In this respect, use of an alcohol-based solvent can solve the problem of the paste-like product. However, a primary alcohol such as methanol or ethanol, when used as a solvent of hydrogenation reaction, has a partial pressure due to the influence of a high reaction temperature of hydrogenation reaction and requires a long reaction time because a hydrogen gas concentration in a gas phase is lowered. Alternatively, use of a cyclic alcohol cyclohexanol can solve the problem of the paste-like product and the problem of the lowered hydrogen gas concentration in a gas phase. However, the present inventors have found that this solvent partially causes transesterification reaction with an ester compound and approximately 1% of the transesterification reaction product tends to remain in the hydrogenation reaction liquid. In contrast to these, use of the secondary alcohol and/or tertiary alcohol, either of which is linear or branched, as the solvent in hydrogenation can solve the problem of the paste-like product, the problem of the lowered hydrogen gas concentration in a gas phase, and the problem of 1% or more of the transesterification reaction product remaining.

Examples of the secondary alcohol and/or tertiary alcohol, either of which is linear or branched, include, but are not particularly limited to: secondary alcohols such as 2-octanol, 2-nonanol, 2-decanol, 4-decanol, 2-undecanol, 3-undecanol, 4-undecanol, 5-undecanol, 2-dodecanol, 2-tridecanol, and 2-tetradecanol; and tertiary alcohols such as tetrahydrolinalool, 3-methyl-3-heptanol, 3-ethyl-3-pentanol, 4-methyl-4-heptanol, 3,4-dimethyl-3-hexanol, 3,5-dimethyl-3-hexanol, and 3-ethyl-2-methyl-3-pentanol.

In the present embodiment, the boiling point of the solvent is preferably 100 to 230°C, more preferably 140 to 220°C, further preferably 160 to 200°C, from the viewpoint of more effectively suppressing the production of the intermediate and the production of the by-product. The boiling point described above adopts a value known as the boiling point of the solvent of interest at normal temperature and pressure.

In the present embodiment, the solvent is preferably represented by the following formula (a) from the viewpoint of more effectively suppressing the production of the intermediate and the production of the by-product: 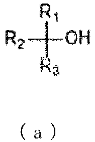 wherein R₁ represents H or CH₃, R₂ represents CH₃, C₂H₅, C₃H₇, or C₄H₉, and R₃ represents C₃H₇, C₄H₉, C₅H₁₁, C₆H₁₃, C₇H₁₅, C₈H₁₇, C₉H₁₉, C₁₀H₂₁, C₁₁H₂₃, or C₁₂H₂₅.

Among those described above, solvents such as 2-octanol and tetrahydrolinalool are especially preferred.

Examples of the hydrogenation catalyst for use in the step (A) include, but are not limited to, catalysts containing at least one element selected from the group consisting of copper, chromium, iron, zinc, and aluminum. Among them, copper-based catalysts such as a Cu-Cr catalyst, a Cu-Zn catalyst, a Cu-Zn-Al catalyst, and a Cu-Fe-Al catalyst are preferred, and a Cu-Cr catalyst and a Cu-Zn-Al catalyst are more preferred.

The amount of the hydrogenation catalyst used is not particularly limited and is preferably 1 to 50% by mass based on the starting compound represented by the formula (2) which is a substrate. When the amount of the hydrogenation catalyst used is 1% by mass or more, the reaction proceeds sufficiently. As a result, the yield of the target compound tends to be improved. Even if the amount of the hydrogenation catalyst used is more than 50% by mass, a reaction rate improving effect commensurate with the amount of the catalyst subjected to the reaction tends to be not obtained. Hence, it is preferred from an economic standpoint to set the amount of the hydrogenation catalyst used to 50% by mass or less. Specifically, when the amount of the hydrogenation catalyst used falls within the range mentioned above, the hydrogenation reaction tends to be able to be efficiently carried out. From the viewpoint described above, the amount of the hydrogenation catalyst used is more preferably 2 to 20% by mass, further preferably 5 to 10% by mass.

In the present embodiment, it is preferred that in the step (A), the catalyst should be activated at a first temperature and a first pressure, and after the activation, the starting compound should be hydrogenated at a second temperature and a second pressure, from the viewpoint of more enhancing reaction efficiency. As mentioned above, a by-product water probably inactivates the activated hydrogenation catalyst. It is therefore preferred to perform the removal of water before hydrogenation.

The activation of the hydrogenation catalyst is an operation for reducing the hydrogenation catalyst and can be carried out at a first temperature and a first pressure.

In the present embodiment, the first temperature is preferably 100°C or higher and 170°C or lower. When the first temperature is 170°C or lower, side reaction or decomposition reaction tends to be prevented. When the first temperature is 100°C or higher, the reduction reaction of the catalyst tends to be completed in a moderate time. From a similar viewpoint, the first temperature is more preferably 120 or higher and 170°C or lower.

In the present embodiment, the first pressure is preferably 0.5 MPa or higher and 5 MPa or lower. When the first pressure is 0.5 MPa or higher, a sufficient reaction rate tends to be obtained. When the first pressure is 5 MPa or lower, a loss of a H₂ gas is reduced in removing water. This tends to be economically advantageous. From a similar viewpoint, the first pressure is more preferably 1 MPa or higher and 3 MPa or lower, further preferably 1.5 MPa or higher and 2 MPa or lower.

In the present embodiment, the reaction time for the activation of the hydrogenation catalyst is preferably 0.2 hours or longer and 3 hours or shorter. When the first reaction time is 0.2 hours or longer, the reduced state of the catalyst tends to be more favorable. When the first reaction time is 3 hours or shorter, the reaction time tends to be able to be decreased while the reduction reaction of the catalyst is allowed to proceed sufficiently.

The activation of the hydrogenation catalyst may be carried out in the absence of the starting compound. In the present embodiment, it is preferred to carry out the activation in the presence of the starting compound, from the viewpoint of reaction efficiency. In the case of carrying out the activation of the hydrogenation catalyst in the presence of the starting compound, water is produced as a by-product reduced water. An operation for removing such water is not particularly limited, and, for example, any of the following methods can be used: a method of discharging water present in a gas-phase moiety to the outside of the reaction system by purging the gas-phase moiety several times; and a method of discharging water to the outside of the reaction system by placing a nozzle line in the mixed liquid in advance and bubbling a H₂ gas or an inert gas such as N₂. The completion of the treatment can be determined by using a method of placing an exhaust gas trap and continuing an operation with two to three times the expected amount of water as a guideline, or a method of measuring a water content value in a reaction liquid.

The hydrogenation of the starting compound is an operation for obtaining the target compound and can be carried out at a second temperature and a second pressure.

In the present embodiment, the second temperature is preferably higher than 170°C and 250°C or lower. When the second temperature is 250°C or lower, the target compound tends to be obtained at a high yield by preventing side reaction or decomposition reaction. When the second temperature is higher than 170°C, the reaction tends to be completed in a moderate time. Thus, reduction in productivity or reduction in target compound yield tends to be able to be circumvented. From a similar viewpoint, the second temperature is more preferably 190°C or higher and 230°C or lower.

In the present embodiment, the second pressure is preferably higher than 5 MPa and 20 MPa or lower. When the second pressure is 20 MPa or lower, the target compound tends to be obtained at a high yield by preventing side reaction or decomposition reaction. When the second pressure is higher than 5 MPa, the reaction tends to be completed in a moderate time. From a similar viewpoint, the second pressure is more preferably higher than 5 MPa and 15 MPa or lower, further preferably higher than 5 MPa and 12 MPa or lower.

The hydrogenation can be preferably carried out at a lower pressure in view of the design of a reaction apparatus (facility cost). From such a viewpoint, the second pressure is preferably 8 MPa or lower, more preferably higher than 5 MPa and 8 MPa or lower. Such conditions tend to lengthen a reaction time. In this context, the amount of the intermediate or the by-product tends to increase if the reaction temperature is elevated in order to shorten the reaction time. Even in such a case, use of the solvent according to the present embodiment can significantly reduce the production of the intermediate and the by-product. In the present embodiment, it is preferred to use a linear or branched tertiary alcohol as the solvent in the step (A), and it is especially preferred to use tetrahydrolinalool as the solvent, from the viewpoint of more effectively reducing the production of the intermediate and the by-product at the second pressure set to 8 MPa or lower. From the viewpoint mentioned above, in the case of using tetrahydrolinalool as the solvent in the present embodiment, it is preferred that the second pressure should be higher than 5 MPa and 8 MPa or lower and the second temperature should be 215°C or higher and 250°C or lower. In the case of using tetrahydrolinalool as the solvent, it is more preferred that the second pressure should be higher than 5 MPa and 8 MPa or lower and the second temperature should be 220°C or higher and 250°C or lower, from the viewpoint of more shortening the reaction time.

In the hydrogenation according to the present embodiment, a gas inert to the hydrogenation reaction (e.g., nitrogen or argon) may be allowed to coexist.

The pressures mentioned above mean values as hydrogen partial pressures.

In the present embodiment, the content of the intermediate represented by the formula (3) in the target compound obtained in the step (A) according to the present embodiment is preferably 0.5% by mass or less, from the viewpoint of physical properties to be obtained when the target compound is further processed. The content of the intermediate can be measured on the basis of a method described in Examples mentioned later. The content of the intermediate can be adjusted to the range described above, for example, by using the secondary alcohol and/or tertiary alcohol, either of which is linear or branched, in the step (A).

In the present embodiment, the content of the by-product represented by the formula (4) in the target compound obtained in the step (A) according to the present embodiment is preferably 0.5% by mass or less, from the viewpoint of physical properties to be obtained when the target compound is further processed. The content of the intermediate can be measured on the basis of a method described in Examples mentioned later. The content of the intermediate can be adjusted to the range described above, for example, by using the linear or branched secondary alcohol and/or tertiary alcohol in the step (A).

The target compound obtained in the step (A) according to the present embodiment can be purified by, for example, distillation, extraction, or crystal precipitation.

### Examples

Hereinafter, the present embodiment will be described in more detail with reference to Examples. However, the present embodiment is not limited by these Examples.

### <Analysis method>

(1) Measurement conditions for gas chromatography analysis
   Analyzer: Capillary gas chromatograph GC-2010 Plus manufactured by Shimadzu Corp.
   Analysis column 1: InertCap 1 (30 m, 0.32 mm I.D.,
   thickness: 0.25 µm) manufactured by GL Sciences Inc.
   Oven temperature 1: 60°C (0.5 min)-15°C/min-280°C (4 min)
   Detector 1: FID, temperature: 280°C
   Internal standard: p-Xylene
   Analysis column 2: InertCap WAX (30 m, 0.32 mm I.D.,
   thickness: 0.25 µm) manufactured by GL Sciences Inc.
   Oven temperature 2: 60°C (0.5 min)-20°C/min-250°C (20 min)
   Detector 2: FID, temperature: 250°C
(2) GC-MS measurement conditions
   Analyzer: GCMS-QP2010 Plus manufactured by Shimadzu Corp.
   Ionization voltage: 70 V
   Analysis column: DB-1 (30 m, 0.32 mm I.D., thickness: 1.00 µm) manufactured by Agilent Technologies, Inc.
   Oven temperature: 60°C (0.5 min)-15°C/min-280°C (4 min)

### <Example 1>

### (Obtainment of monoolefin)

215 g (2.50 mol) of methyl acrylate and 165 g (1.25 mol) of dicyclopentadiene were charged into a 500 mL stainless reactor, and were reacted at 220°C for 2 hours. A reaction liquid containing 152 g of monoolefin represented by the following formula (5a) was obtained and subjected to distillation purification. Then, a portion of the purified product was used for the subsequent reaction.

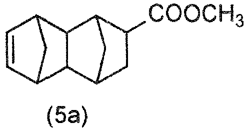

### (Obtainment of aldehyde)

A 500 mL stainless reactor was used to perform the hydroformylation reaction of the monoolefin represented by the formula (5a). To the reactor were added 100 g of the distillation-purified monoolefin represented by the formula (5a), 96 g of 2-octanol (manufactured by Ogura Gosei Kogyo Ltd.), 213 mg (1500 ppm by mol based on the monoolefin) of triphenyl phosphite (manufactured by FUJIFILM Wako Pure Chemical Corp.), and 355 µg (3 ppm by mol based on the monoolefin) of Rh(acac)(CO)₂ (manufactured by N.E. CHEMCAT Corp.). The atmosphere inside the reactor was replaced with nitrogen and a CO/H₂ mixed gas three times each. Then, the system was pressurized with a CO/H₂ mixed gas, followed by reaction at 100°C at 2 MPa for 3 hours.

After the completion of reaction, the reaction liquid was analyzed by gas chromatography and GC-MS. As a result, a reaction liquid containing 112.6 g of a starting compound represented by the following formula (2a) having a molecular weight of 248 (rate of substrate conversion: 100% (= (Monoolefin charged - Remaining monoolefin charged) / Monoolefin charged) × 100%) was obtained as a main product with an aldehyde yield of 99.5% (= Main product / Monoolefin charged × 100%)).

Subsequently, the starting compound represented by the formula (2a) was subjected to distillation purification. Then, a portion of the purified product was subjected to the subsequent reaction.

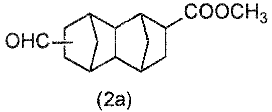

### (Reduction of catalyst)

To a 300 mL stainless reactor equipped with a nozzle for nitrogen bubbling were added 54.94 g of the distillation-purified starting compound represented by the formula (2a), 8.24 g of a Cu-Zn-Al catalyst (E-01X manufactured by JGC C&C), and 120.86 g of 2-octanol_ (manufactured by Ogura Gosei Kogyo Ltd.). The atmosphere inside the reactor was replaced with nitrogen and a H₂ gas three times each. Then, the system was pressurized with a H₂ gas, followed by the reduction of the catalyst at 150°C at 2 MPa for 1 hour. A trap was placed in an exhaust gas line. While the temperature was kept at 150°C, the pressure was dropped to 0 MPa. Then, a very small amount of a nitrogen gas was injected from the nozzle for nitrogen bubbling to remove reduced water in the reaction liquid. The amount of a liquid trapped was 3.90 g.

### (Obtainment of dimethanol through hydrogenation reaction)

Subsequently, the atmosphere inside the reactor was replaced with a H₂ gas three times. Then, the system was pressurized with a H₂ gas, followed by hydrogenation reaction at 215°C at 9.5 MPa. Specifically, the hydrogenation reaction solvent used was 2-octanol. During the reaction, sampling was performed every 5 hours.

The sampled liquid was diluted with acetone, and the catalyst was filtered off through a membrane filter having a pore size of 0.2 µm. Then, a target compound represented by the formula (1a) given below having a molecular weight of 222 as a main product as well as an ester compound (intermediate) represented by the formula (3a) given below having a molecular weight of 250, and a transesterified compound (by-product) represented by the formula (4a) given below having a molecular weight of 349 were quantitatively analyzed by gas chromatography and GC-MS. This quantitative analysis was conducted using analysis column 2 (InertCap WAX). The amount of the intermediate represented by the formula (3a) 15 hours later was 0.45% (= Intermediate represented by the formula (3a) / (All the components except for 2-octanol and acetone) × 100). The amount of the by-product represented by the formula (4a) was 0.42% (= By-product represented by the formula (4a) / (All the components except for 2-octanol and acetone) × 100).

After the completion of reaction, the reaction liquid was analyzed by gas chromatography using analysis column 1 (InertCap 1) to confirm a reaction liquid containing 47.46 g of the target compound represented by the formula (1a) (rate of substrate conversion: 100% (= (Starting compound charged - Remaining starting compound) / Starting compound charged) × 100%)) with a target compound yield of 96.50% (= Target compound / Starting compound charged × 100%)).

Further, the reaction liquid was subjected to simple distillation purification using a 300 mL three-neck recovery flask equipped with a thermometer, a nozzle for N₂ bubbling, and a stirring bar (the number of distillation stages: one stage, distillate temperature: 170 to 180°C, degree of vacuum: 0.27 kPa (2 torr)). The purified product was analyzed by gas chromatography using analysis column 1 (InertCap 1) to confirm 44.03 g of a main fraction containing the target compound represented by the formula (1a) with a dimethanol purity of 99.17% (= Target compound represented by the formula (1a) / (All the components except for acetone) × 100)) and a distillation yield of 92.00% (=Target compound obtained / Target compound charged × 100%)). Also, analysis was conducted using analysis column 2 (InertCap WAX). The amount of the intermediate represented by the formula (3a) was 0.43% (= Intermediate represented by the formula (3a) / (All the components except for 2-octanol and acetone) × 100). The amount of the by-product represented by the formula (4a) was 0.40% (= By-product represented by the formula (4a) / (All the components except for 2-octanol and acetone) × 100).

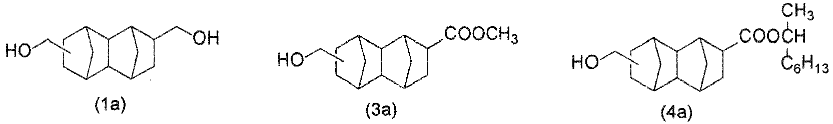

### <Comparative Example 1>

Hydrogenation reaction was carried out in the same manner as in Example 1 except that 1-octanol was used instead of 2-octanol as the hydrogenation reaction solvent. The amount of the intermediate represented by the formula (3a) 15 hours later was 0.47% (= Intermediate represented by the formula (3a) / (All the components except for 2-octanol and acetone) × 100). The amount of a by-product represented by the formula (4b) was 4.23% (= By-product represented by the formula (4b) / (All the components except for 2-octanol and acetone) × 100).

After the completion of reaction, the reaction liquid was analyzed by gas chromatography using analysis column 1 (InertCap 1) to confirm a reaction liquid containing 45.58 g of the target compound represented by the formula (1a) (rate of substrate conversion: 100% (= (Starting compound charged - Remaining starting compound) / Starting compound charged) × 100%) with a dimethanol yield of 92.67% (= Target compound / Starting compound charged × 100%)).

Further, the reaction liquid was subjected to simple distillation purification using a 300 mL three-neck recovery flask equipped with a thermometer, a nozzle for N₂ bubbling, and a stirring bar (the number of distillation stages: one stage, distillate temperature: 170 to 180°C, degree of vacuum: 0.27 kPa (2 torr)). The purified product was analyzed by gas chromatography using analysis column 1 (InertCap 1). As a result, 40.49 g of a main fraction containing the target compound represented by the formula (1a) was confirmed with a dimethanol purity of 98.28% (= Target compound represented by the formula (1a) / (All the components except for acetone) × 100)) and a distillation yield of 87.30% (= Target compound obtained / Target compound charged × 100%)). Also, analysis was conducted using analysis column 2 (InertCap WAX). The amount of the intermediate represented by the formula (3a) was 0.45% (= Intermediate represented by the formula (3a) / (All the components except for 2-octanol and acetone) × 100). The amount of the by-product represented by the formula (4b) was 1.27% (= By-product represented by the formula (4b) / (All the components except for 2-octanol and acetone) × 100).

Since the solvent according to the present embodiment was not used, the hydrogenation yield was low. Furthermore, the content of the by-product represented by the formula (4b) was large, resulting in a low product purity.

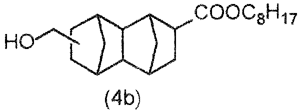

### <Comparative Example 2>

Hydrogenation reaction was carried out in the same manner as in Example 1 except that 2-ethyl-hexanol was used instead of 2-octanol as the hydrogenation reaction solvent. The amount of the intermediate represented by the formula (3a) 15 hours later was 0.30% (= Intermediate represented by the formula (3a) / (All the components except for 2-octanol and acetone) × 100). The amount of a by-product represented by the formula (4c) was 3.93% (= By-product represented by the formula (4c) / (All the components except for 2-octanol and acetone) × 100).

After the completion of reaction, the reaction liquid was analyzed by gas chromatography using analysis column 1 (InertCap 1) to confirm a reaction liquid containing 45.81 g of the target compound represented by the formula (1a) (rate of substrate conversion: 100% (= Starting compound charged - Remaining starting compound) / Starting compound charged) × 100%) with a dimethanol yield of 93.14% (= Target compound / Starting compound charged × 100%)).

Further, the reaction liquid was subjected to simple distillation purification using a 300 mL three-neck recovery flask equipped with a thermometer, a nozzle for N₂ bubbling, and a stirring bar (the number of distillation stages: one stage, distillate temperature: 170 to 180°C, degree of vacuum: 0.27 kPa (2 torr)). The purified product was analyzed by gas chromatography using analysis column 1 (InertCap 1). As a result, 40.85 g of a main fraction containing the target compound represented by the formula (1a) was confirmed with a dimethanol purity of 98.46% (= Target compound represented by the formula (1a) / (All the components except for acetone) × 100)) and a distillation yield of 87.80% (= Target compound obtained / Target compound charged × 100%)). Also, analysis was conducted using analysis column 2 (InertCap WAX). The amount of the intermediate represented by the formula (3a) was 0.29% (= Intermediate represented by the formula (3a) / (All the components except for 2-octanol and acetone) × 100). The amount of the by-product represented by the formula (4c) was 1.26% (= By-product represented by the formula (4c) / (All the components except for 2-octanol and acetone) × 100).

Since the solvent according to the present embodiment was not used, the hydrogenation yield was low. Furthermore, the content of the by-product represented by the formula (4c) was large, resulting in a low product purity.

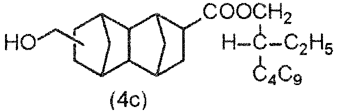

### <Example 2>

Hydrogenation reaction was carried out in the same manner as in Example 1 except that tetrahydrolinalool was used instead of 2-octanol as the hydrogenation reaction solvent. The amount of the intermediate represented by the formula (3a) 15 hours later was 0.35% (= Intermediate represented by the formula (3a) / (All the components except for 2-octanol and acetone) × 100). The amount of a by-product represented by the formula (4d) was 0% (= By-product represented by the formula (4d) / (All the components except for 2-octanol and acetone) × 100).

After the completion of reaction, the reaction liquid was analyzed by gas chromatography using analysis column 1 (InertCap 1) to confirm a reaction liquid containing 48.11 g of the target compound represented by the formula (1a) (rate of substrate conversion: 100% (= Starting compound charged - Remaining starting compound) / Starting compound charged) × 100%) with a dimethanol yield of 97.82% (= Target compound / Starting compound charged × 100%)).

Further, the reaction liquid was subjected to simple distillation purification using a 300 mL three-neck recovery flask equipped with a thermometer, a nozzle for N₂ bubbling, and a stirring bar (the number of distillation stages: one stage, distillate temperature: 170 to 180°C, degree of vacuum: 0.27 kPa (2 torr)). The purified product was analyzed by gas chromatography using analysis column 1 (InertCap 1) to confirm 44.99 g of a main fraction containing the target compound represented by the formula (1a) with a dimethanol purity of 99.67% (= Target compound represented by the formula (1a) / (All the components except for acetone) × 100)) and a distillation yield of 93.20% (=Target compound obtained / Target compound charged × 100%)). Also, analysis was conducted using analysis column 2 (InertCap WAX). The amount of the intermediate represented by the formula (3a) was 0.33% (= Intermediate represented by the formula (3a) / (All the components except for 2-octanol and acetone) × 100). The amount of the by-product represented by the formula (4d) was 0.00% (= By-product represented by the formula (4d) / (All the components except for 2-octanol and acetone) × 100).

Since the solvent according to the present embodiment was used, the hydrogenation yield was high. Furthermore, the content of the by-product represented by the formula (4d) was small, resulting in a high product purity.

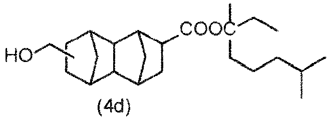

### <Comparative Example 3>

Hydrogenation reaction was carried out in the same manner as in Example 1 except that cyclohexanol was used instead of 2-octanol as the hydrogenation reaction solvent. The amount of the intermediate represented by the formula (3a) 15 hours later was 0.50% (= Intermediate represented by the formula (3a) / (All the components except for 2-octanol and acetone) × 100). The amount of a by-product represented by the formula (4e) was 0.96% (= By-product represented by the formula (4e) / (All the components except for 2-octanol and acetone) × 100).

After the completion of reaction, the reaction liquid was analyzed by gas chromatography using analysis column 1 (InertCap 1) to confirm a reaction liquid containing 47.17 g of the target compound represented by the formula (1a) (rate of substrate conversion: 100% (= (Starting compound charged - Remaining starting compound) / Starting compound charged) × 100%) with a dimethanol yield of 95.91% (= Target compound / Starting compound charged × 100%)).

Further, the reaction liquid was subjected to simple distillation purification using a 300 mL three-neck recovery flask equipped with a thermometer, a nozzle for N₂ bubbling, and a stirring bar (the number of distillation stages: one stage, distillate temperature: 170 to 180°C, degree of vacuum: 0.27 kPa (2 torr)). The purified product was analyzed by gas chromatography using analysis column 1 (InertCap 1) to confirm 43.76 g of a main fraction containing the target compound represented by the formula (1a) with a dimethanol purity of 98.64% (= Target compound represented by the formula (1a) / (All the components except for acetone) × 100)) and a distillation yield of 91.50% (= Target compound obtained / Target compound charged × 100%)). Also, analysis was conducted using analysis column 2 (InertCap WAX). The amount of the intermediate represented by the formula (3a) was 0.45% (= Intermediate represented by the formula (3a) / (All the components except for 2-octanol and acetone) × 100). The amount of the by-product represented by the formula (4e) was 0.91% (= By-product represented by the formula (4e) / (All the components except for 2-octanol and acetone) × 100).

Since the solvent according to the present embodiment was not used, the hydrogenation yield was low. Furthermore, the content of the by-product represented by the formula (4e) was large, resulting in a low product purity.

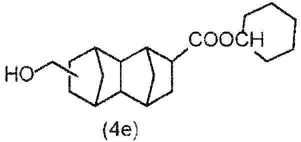

The reaction conditions, the reaction results, etc. of Examples 1 and 2 and Comparative Examples 1 to 3 are shown in Table 1.

**[Table 1]**

| | | | Example 1 | | Comparative Example 1 | | Comparative Example 2 | | Example 2 | | Comparative Example 3 | |
|---|---|---|---|---|---|---|---|---|---|---|---|---|
| Solvent | Solvent species | | 2-Octanol | | 1-Octanol | | 2-Ethylhexanol | | Tetrahydrolinalool | | Cyclohexanol | |
| | Structure | | 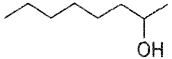 | | 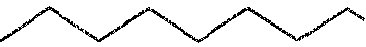 | | 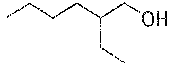 | | 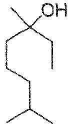 | | 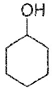 | |
| | Melting point | (°C) | -38 | | -17 | | -76 | | -56 | | 23 | |
| | Boiling point | (°C) | 179 | | 194 | | 185 | | 196 | | 161 | |
| Hydrogenation reaction conditions | Temperature | (°C) | 215 | | 215 | | 215 | | 215 | | 215 | |
| | Pressure | (MPa) | 10 | | 10 | | 10 | | 10 | | 10 | |
| | Starting compound (formula (2a)) | (9) | 54.94 | | 54.94 | | 54.94 | | 54.94 | | 54.94 | |
| | Amount of catalyst (E01-X) | (9) | 8.24 | | 8.24 | | 8.24 | | 8.24 | | 8.24 | |
| | Catalyst/substrate | (wt%) | 15.00 | | 15.00 | | 15.00 | | 15.00 | | 15.00 | |
| | Amount of solvent | (g) | 120.86 | | 120.86 | | 120.86 | | 120.86 | | 120.86 | |
| | Amount of liquid trapped | (g) | 3.90 | | 2.29 | | 4.94 | | 3.19 | | 4.50 | |
| Hydrogenation reaction results | GC analysis | | Intermediate (formula (3a)) | By-product (formula 4(a)) | Intermediate (formula (3a)) | By-product (formula 4(b)) | Intermediate (formula (3a)) | By-product (formula 4(c)) | Intermediate (formula (3a)) | By-product (formula 4(d)) | Intermediate (formula (3a)) | By-product (formula 4(e)) |
| | | 5hr Later | 7.54% | 0.22% | 5.87% | 5.16% | 4.78% | 2.84% | 3.06% | 0% | 3.34% | 0.75% |
| | | 10hr Later | 1.14% | 0.33% | 1.07% | 4.88% | 0.87% | 3.68% | 0.66% | 0% | 0.74% | 0.83% |
| | | 15hr Later | 0.45% | 0.42% | 0.47% | 4.23% | 0.30% | 3.93% | 0.35% | 0% | 0.50% | 0.96% |
| | Amount of target compound (formula (1a)) | (g) | 47.46 | | 45.58 | | 45.81 | | 48.11 | | 47.17 | |
| | Hydrogenation yield | (mol%) | 96.50 | | 92.67 | | 93.14 | | 97,82 | | 95.91 | |
| Distillation | Main fraction | (g) | 44.03 | | 40.49 | | 40.85 | | 44.99 | | 43.76 | |
| | Purity | (GC%) | 99.17 | | 98.28 | | 98.46 | | 99.67 | | 98.64 | |
| | Distillation yield | (mol%) | 92.00 | | 87.30 | | 87.80 | | 93.20 | | 91.50 | |
| | Amount of impurities after distillation | | Intermediate (formula (3a)) | By-product (formula 4(a)) | Intermediate (formula (3a)) | By-product (formula 4(b)) | Intermediate (formula (3a)) | By-product (formula 4(c)) | Intermediate (formula (3a)) | By-product (formula 4(d)) | Intermediate (formula (3a)) | By-product (formula 4(e)) |
| | | (GC%) | 0.43 | *0.40* | 0.45 | 1.27 | 0.29 | 1.26 | 0.33 | 0.00 | 0.45 | 0.91 |

### <Example 3>

### (Obtainment of aldehyde)

A 500 mL stainless reactor was used to perform the hydroformylation reaction of the monoolefin represented by the formula (5a). To the reactor were added 100 g of the distillation-purified monoolefin represented by the formula (5a), 96 g of 2-octanol (manufactured by Ogura Gosei Kogyo Ltd.), 213 mg (1500 ppm by mol based on the monoolefin) of triphenyl phosphite (manufactured by FUJIFILM Wako Pure Chemical Corp.), and 355 µg (3 ppm by mol based on the monoolefin) of Rh(acac)(CO)₂ (manufactured by N.E. CHEMCAT Corp.). The atmosphere inside the reactor was replaced with nitrogen and a CO/H₂ mixed gas three times each. Then, the system was pressurized with a CO/H₂ mixed gas, followed by reaction at 100°C at 2 MPa for 3 hours.

After the completion of reaction, the reaction liquid was analyzed by gas chromatography and GC-MS. As a result, a reaction liquid containing 112.6 g of a starting compound represented by the formula (2a) (rate of substrate conversion: 100% (= (Monoolefin charged - Remaining monoolefin charged) / Monoolefin charged) × 100%) was obtained as a main product with an aldehyde yield of 99.5% (= Main product / Monoolefin charged × 100%)).

Subsequently, the starting compound represented by the formula (2a) was subjected to distillation purification. Then, a portion of the purified product was subjected to the subsequent reaction.

### (Reduction of catalyst)

To a 300 mL stainless reactor equipped with a nozzle for nitrogen bubbling were added 54.94 g of the distillation-purified starting compound represented by the formula (2a), 8.24 g of a Cu-Zn-Al catalyst (E-01X manufactured by JGC C&C), and 120.86 g of tetrahydrolinalool (manufactured by Tokyo Chemical Industry Co., Ltd.). The atmosphere inside the reactor was replaced with nitrogen and a H₂ gas three times each. Then, the system was pressurized with a H₂ gas, followed by the reduction of the catalyst at 150°C at 2 MPa for 1 hour. A trap was placed in an exhaust gas line. While the temperature was kept at 150°C, the pressure was dropped to 0 MPa. Then, a very small amount of a nitrogen gas was injected from the nozzle for nitrogen bubbling to remove reduced water in the reaction liquid. The amount of a liquid trapped was 6.29 g.

### (Obtainment of dimethanol through hydrogenation reaction)

Subsequently, the atmosphere inside the reactor was replaced with a H₂ gas three times. Then, the system was pressurized with a H₂ gas, followed by hydrogenation reaction at 215°C at 8.0 MPa. Specifically, the hydrogenation reaction solvent used was tetrahydrolinalool. During the reaction, sampling was performed every 5 hours or every 10 hours.

The sampled liquid was diluted with acetone, and the catalyst was filtered off through a membrane filter having a pore size of 0.2 µm. Then, a target compound represented by the formula (1a) as a main product as well as an ester compound (intermediate) represented by the formula (3a), and a transesterified compound (by-product) represented by the formula (4d) having a molecular weight of 378 were quantitatively analyzed by gas chromatography and GC-MS. This quantitative analysis was conducted using analysis column 2 (InertCap WAX). The amount of the intermediate represented by the formula (3a) 50 hours later was 0.50% (= Intermediate represented by the formula (3a) / (All the components except for tetrahydrolinalool and acetone) × 100). The amount of the by-product represented by the formula (4d) was 0.00% (= By-product represented by the formula (4d) / (All the components except for tetrahydrolinalool and acetone) × 100).

After the completion of reaction, the reaction liquid was analyzed by gas chromatography using analysis column 1 (InertCap 1) to confirm a reaction liquid containing 47.96 g of the target compound represented by the formula (1a) (rate of substrate conversion: 100% (= (Starting compound charged - Remaining starting compound) / Starting compound charged) × 100%) with a target compound yield of 97.50% (= Target compound / Starting compound charged × 100%)).

Further, the reaction liquid was subjected to simple distillation purification using a 300 mL three-neck recovery flask equipped with a thermometer, a nozzle for nitrogen bubbling, and a stirring bar (the number of distillation stages: one stage, distillate temperature: 170 to 180°C, degree of vacuum: 0.27 kPa (2 torr)). The purified product was analyzed by gas chromatography using analysis column 1 (InertCap 1) to confirm 45.05 g of a main fraction containing the target compound represented by the formula (1a) with a dimethanol purity of 99.53% (= Target compound represented by the formula (1a) / (All the components except for acetone) × 100)) and a distillation yield of 93.50% (= Target compound obtained / Target compound charged × 100%)). Also, analysis was conducted using analysis column 2 (InertCap WAX). The amount of the intermediate represented by the formula (3a) was 0.48% (= Intermediate represented by the formula (3a) / (All the components except for acetone) × 100). The amount of the by-product represented by the formula (4d) was 0.00% (= By-product represented by the formula (4d) / (All the components except for acetone) × 100).

### <Example 4>

Hydrogenation reaction was carried out in the same manner as in Example 3 except that: the amount of a liquid trapped in the reduction of the catalyst was adjusted to 5.22 g; and the hydrogenation reaction temperature was set to 225°C. The amount of the intermediate represented by the formula (3a) 20 hours later was 0.66% (= Intermediate represented by the formula (3a) / (All the components except for tetrahydrolinalool and acetone) × 100). The amount of the by-product represented by the formula (4d) was 0% (= By-product represented by the formula (4d) / (All the components except for tetrahydrolinalool and acetone) × 100).

After the completion of reaction, the reaction liquid was analyzed by gas chromatography using analysis column 1 (InertCap 1) to confirm a reaction liquid containing 47.88 g of the target compound represented by the formula (1a) (rate of substrate conversion: 100% (= (Starting compound charged - Remaining starting compound) / Starting compound charged) × 100%) with a dimethanol yield of 97.34% (= Target compound / Starting compound charged × 100%)).

Further, the reaction liquid was subjected to simple distillation purification using a 300 ml three-neck recovery flask equipped with a thermometer, a nozzle for N₂ bubbling, and a stirring bar (the number of distillation stages: one stage, distillate temperature: 170 to 180°C, degree of vacuum: 0.27 kPa (2 torr)). The purified product was analyzed by gas chromatography using analysis column 1 (InertCap 1). As a result, 43.64 g of a main fraction containing the target compound represented by the formula (1a) was confirmed with a dimethanol purity of 99.51% (= Target compound represented by the formula (1a) / (All the components except for acetone) × 100)) and a distillation yield of 90.70% (= Target compound obtained / Target compound charged × 100%)). Also, analysis was conducted using analysis column 2 (InertCap WAX). The amount of the intermediate represented by the formula (3a) was 0.50% (= Intermediate represented by the formula (3a) / (All the components except for acetone) × 100). The amount of the by-product represented by the formula (4d) was 0.00% (= By-product represented by the formula (4d) / (All the components except for acetone) × 100).

Since the hydrogenation reaction temperature was elevated, the reaction rate was accelerated and productivity was able to be improved. Furthermore, the by-product represented by the formula (4d) was confirmed to be absent, resulting in a high product purity.

The reaction conditions, the reaction results, etc. of Examples 3 and 4 are shown in Table 2.

**[Table 2]**

| | | | Example 3 | | Example 4 | |
|---|---|---|---|---|---|---|
| Solvent | Solvent species | | Tetrahydrolinalool | | Tetrahydrolinalool | |
| | Structure | | 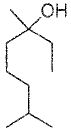 | | 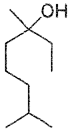 | |
| | Melting point | (°C) | -56 | | -56 | |
| | Boiling point | (°C) | 196 | | 196 | |
| Hydrogenation reaction conditions | Temperature | (°C) | 215 | | 225 | |
| | Pressure | (MPa) | 8 | | 8 | |
| | Starting compound (formula (2a)) | (9) | 54.94 | | 54.94 | |
| | Amount of catalyst (E01-X) | (g) | 8.24 | | 8.24 | |
| | Catalyst/substrate | (wt%) | 15.00 | | 15.00 | |
| | Amount of solvent | (g) | 120.86 | | 120.86 | |
| | Amount of liquid trapped | (g) | 6.29 | | 5.22 | |
| Hydrogenation reaction results | GC analysis | | Intermediate (formula (3a)) | By-product (formula 4(d)) | Intermediate (formula (3a)) | By-product (formula 4(d)) |
| | | 5hr Later | 13.51% | 0% | 1.98% | 0% |
| | | 10hr Later | 2.28% | 0% | 0.94% | 0% |
| | | 15hr Later | 1.02% | 0% | 0.73% | 0% |
| | | 20hr Later | 0.76% | 0% | 0.66% | 0% |
| | | 30hr Later | 0.61% | 0% | | |
| | | 40hr Later | 0.54% | 0% | | |
| | | 50hr Later | 0.50% | 0% | | |
| | Amount of target compound (formula (1a)) | (g) | 47.96 | | 47.88 | |
| | Hydrogenation yield | (mol%) | 97.50 | | 97.34 | |
| Distillation | Main fraction | (9) | 45.05 | | 43.64 | |
| | Purity | (GC%) | 99.53 | | 99.51 | |
| | Distillation yield | (mol%) | 93.50 | | 90.70 | |
| | Amount of impurities after distillation | | Intermediate (formula (3a)) | By-product (formula 4(d)) | Intermediate (formula (3a)) | By-product (formula 4(d)) |
| | | (GC%) | 0.48 | 0.00 | 0.50 | 0.00 |

The present application claims priority to Japanese Patent Application No. 2021-067275 filed on April 12, 2021.

### Industrial Applicability

The production method according to the present invention can produce a dimethanol compound having a norbornane skeleton which is useful as a paint additive, an adhesive, a resin raw material, or the like by an industrially advantageous method.

## Claims

1. A production method for producing a target compound represented by the following formula (1), the production method comprising:
a step (A) of subjecting a mixed liquid comprising a starting compound represented by the following formula (2) and a solvent to a hydrogenation reduction in a presence of a catalyst having hydrogenation ability,
wherein the solvent is a linear or branched secondary alcohol or tertiary alcohol: 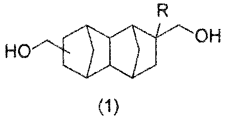 wherein R represents H, CH₃, or C₂H₅, 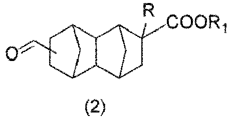 wherein R represents H, CH₃, or C₂H₅, and R₁ represents CH₃, C₂H₅, C₃H₇, or C₄H₉.

2. The production method according to claim 1, wherein a boiling point of the solvent is 100 to 230°C.

3. The production method according to claim 1 or 2, wherein the solvent is represented by the following formula (a): 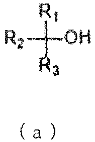 wherein R₁ represents H or CH₃, R₂ represents CH₃, C₂H₅, C₃H₇, or C₄H₉, and R₃ represents C₃H₇, C₄H₉, C₅H₁₁, C₆H₁₃, C₇H₁₅, C₈H₁₇, C₉H₁₉, C₁₀H₂₁, C₁₁H₂₃, or C₁₂H₂₅.

4. The production method according to any one of claims 1 to 3, wherein a content of an intermediate represented by the following formula (3) in the target compound is 0.5% by mass or less: 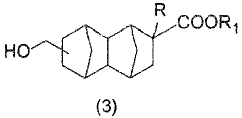 wherein R represents H, CH₃, or C₂H₅, and R₁ represents CH₃, C₂H₅, C₃H₇, or C₄H₉.

5. The production method according to any one of claims 1 to 4, wherein a content of a by-product represented by the following formula (4) in the target compound is 0.5% by mass or less: 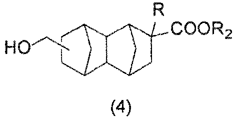 wherein R represents H, CH₃, or C₂H₅, and R₂ represents C₄H₉, C₅H₁₁, C₆H₁₃, C₇H₁₅, C₈H₁₇, C₉H₁₉, C₁₀H₂₁, C₁₁H₂₃, C₁₂H₂₅, C₁₃H₂₇, or C₁₄H₂₉.

6. The production method according to any one of claims 1 to 5, wherein the solvent is 2-octanol and/or tetrahydrolinalool.

7. The production method according to any one of claims 1 to 6, wherein, in the step (A), the catalyst is activated at a first temperature and a first pressure, and after the activation, the starting compound is hydrogenated at a second temperature and a second pressure.

8. The production method according to claim 7, wherein the second pressure is higher than 5 MPa and 20 MPa or lower.

9. The production method according to claim 7 or 8, wherein the second pressure is higher than 5 MPa and 8 MPa or lower.

10. The production method according to claim 9, wherein the solvent is a linear or branched tertiary alcohol.

11. The production method according to claim 10, wherein the solvent is tetrahydrolinalool.

## Patentansprüche

1. Ein Herstellungsverfahren zur Herstellung einer Zielverbindung, dargestellt durch die folgende Formel (1), wobei das Herstellungsverfahren umfasst:
einen Schritt (A) des Unterziehens eines Flüssigkeitsgemischs, das eine Ausgangsverbindung, dargestellt durch die folgende Formel (2), und ein Lösungsmittel umfasst, einer Hydrierungsreduktion in Gegenwart eines Katalysators mit Hydrierungsfähigkeit,
wobei das Lösungsmittel ein linearer oder verzweigter sekundärer Alkohol oder tertiärer Alkohol ist: 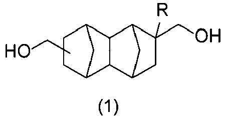
wobei R für H, CH₃ oder C₂H₅ steht, 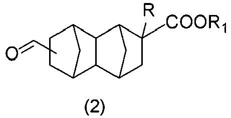
wobei R für H, CH₃ oder C₂H₅ steht und R₁ für CH₃, C₂H₅, C₃H₇ oder C₄H₉ steht.

2. Das Herstellungsverfahren nach Anspruch 1, wobei ein Siedepunkt des Lösungsmittels 100 bis 230°C beträgt.

3. Das Herstellungsverfahren nach Anspruch 1 oder 2, wobei das Lösungsmittel durch die folgende Formel (a) dargestellt ist: 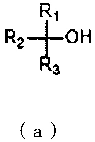 wobei R₁ für H oder CH₃ steht, R₂ für CH₃, C₂H₅, C₃H₇ oder C₄H₉ steht und R₃ für C₃H₇, C₄H₉, C₅H₁₁, C₆H₁₃, C₇H₁₅, C₈H₁₇, C₉H₁₉, C₁₀H₂₁, C₁₁H₂₃ oder C₁₂H₂₅ steht.

4. Das Herstellungsverfahren nach einem der Ansprüche 1 bis 3, wobei ein Gehalt eines durch die folgende Formel (3) dargestellten Zwischenprodukts in der Zielverbindung 0,5 Massen-% oder weniger beträgt: 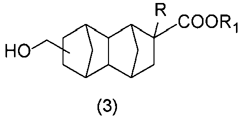 wobei R für H, CH₃ oder C₂H₅ steht und R₁ für CH₃, C₂H₅, C₃H₇ oder C₄H₉ steht.

5. Das Herstellungsverfahren nach einem der Ansprüche 1 bis 4, wobei ein Gehalt eines Nebenprodukts, dargestellt durch die folgende Formel (4), in der Zielverbindung 0,5 Massen-% oder weniger beträgt: 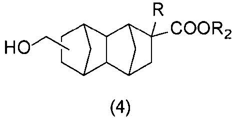 wobei R für H, CH₃ oder C₂H₅ steht und R₂ für C₄H₉, C₅H₁₁, C₆H₁₃, C₇H₁₅, C₈H₁₇, C₉H₁₉, C₁₀H₂₁, C₁₁H₂₃, C₁₂H₂₅, C₁₃H₂₇ oder C₁₄H₂₉ steht.

6. Das Herstellungsverfahren nach einem der Ansprüche 1 bis 5, wobei das Lösungsmittel 2-Octanol und/oder Tetrahydrolinalool ist.

7. Das Herstellungsverfahren nach einem der Ansprüche 1 bis 6, wobei in Schritt (A) der Katalysator bei einer ersten Temperatur und einem ersten Druck aktiviert wird und nach der Aktivierung die Ausgangsverbindung bei einer zweiten Temperatur und einem zweiten Druck hydriert wird.

8. Das Herstellungsverfahren nach Anspruch 7, wobei der zweite Druck höher als 5 MPa und 20 MPa oder niedriger ist.

9. Das Herstellungsverfahren nach Anspruch 7 oder 8, wobei der zweite Druck höher als 5 MPa und 8 MPa oder niedriger ist.

10. Das Herstellungsverfahren nach Anspruch 9, wobei das Lösungsmittel ein linearer oder verzweigter tertiärer Alkohol ist.

11. Das Herstellungsverfahren nach Anspruch 10, wobei das Lösungsmittel Tetrahydrolinalool ist.

## Revendications

1. Méthode de production pour produire un composé cible représenté par la formule suivante (1), la méthode de production comprenant :
une étape (A) de soumission d'un liquide mélangé comprenant un composé de départ représenté par la formule suivante (2) et un solvant à une réduction par hydrogénation en présence d'un catalyseur ayant une aptitude à l'hydrogénation,
dans laquelle le solvant est un alcool secondaire ou un alcool tertiaire linéaire ou ramifié : 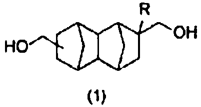
dans laquelle R représente H, CH₃, ou C₂H₅, 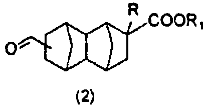
dans laquelle R représente H, CH₃, ou C₂H₅, et R₁ représente CH₃, C₂H₅, C₃H₇ ou C₄H₉.

2. Méthode de production selon la revendication 1, dans laquelle un point d'ébullition du solvant est de 100 à 230°C.

3. Méthode de production selon la revendication 1 ou 2, dans laquelle le solvant est représenté par la formule suivante (a) : 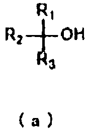 dans laquelle R₁ représente H ou CH₃, R₂ représente CH₃, C₂H₅, C₃H₇, ou C₄H₉, et R₃ représente C₃H₇, C₄H₉, C₅H₁₁, C₆H₁₃, C₇H₁₅, C₈H₁₇, C₉H₁₉, C₁₀H₂₁, C₁₁H₂₃, ou C₁₂H₂₅.

4. Méthode de production selon l'une quelconque des revendications 1 à 3, dans laquelle une teneur en un intermédiaire représenté par la formule suivante (3) dans le composé cible est de 0,5 % en masse ou moins : 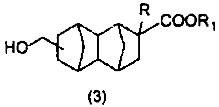 dans laquelle R représente H, CH₃, ou C₂H₅, et R₁ représente CH₃, C₂H₅, C₃H₇, ou C₄H₉.

5. Méthode de production selon l'une quelconque des revendications 1 à 4, dans laquelle une teneur en un sous-produit représenté par la formule suivante (4) dans le composé cible est de 0,5 % en masse ou moins : 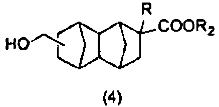 dans laquelle R représente H, CH₃, ou C₂H₅, et R₂ représente C₄H₉, C₅H₁₁, C₆H₁₃, C₇H₁₅, C₈H₁₇, C₉H₁₉, C₁₀H₂₁, C₁₁H₂₃, C₁₂H₂₅, C₁₃H₂₇, ou C₁₄H₂₉.

6. Méthode de production selon l'une quelconque des revendications 1 à 5, dans laquelle le solvant est le 2-octanol et/ou le tétrahydrolinalool.

7. Méthode de production selon l'une quelconque des revendications 1 à 6, dans laquelle, à l'étape (A), le catalyseur est activé à une première température et une première pression, et après activation, le composé de départ est hydrogéné à une deuxième température et une deuxième pression.

8. Méthode de production selon la revendication 7, dans laquelle la deuxième pression est supérieure à 5 MPa et inférieure ou égale à 20 MPa.

9. Méthode de production selon la revendication 7 ou 8, dans laquelle la deuxième pression est supérieure à 5 MPa et inférieure ou égale à 8 MPa.

10. Méthode de production selon la revendication 9, dans laquelle le solvant est un alcool tertiaire linéaire ou ramifié.

11. Méthode de production selon la revendication 10, dans laquelle le solvant est le tétrahydrolinalool.
